(19)

**Europäisches Patentamt**
**European Patent Office**
**Office européen des brevets**

(11) **EP 4 717 238 A1**

(12) **EUROPEAN PATENT APPLICATION**

(43) Date of publication:
01.04.2026 Bulletin 2026/14

(21) Application number: 24203427.0

(22) Date of filing: 30.09.2024

(51) International Patent Classification (IPC):
**A61F 13/01** (2024.01)  **A61F 13/36** (2006.01)
**A61F 15/00** (2006.01)  **A61L 15/24** (2006.01)

(52) Cooperative Patent Classification (CPC):
(C-Sets available)
**A61F 13/36; A61F 13/01017; A61F 13/01029;**
**A61F 13/01042; A61F 15/001; A61L 15/24;**
**A61L 15/425; A61L 15/60**         (Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Mölnlycke Health Care AB**
**431 21 Mölndal (SE)**

(72) Inventors:
• CARLSSON, Erik
**42941 Särö (SE)**
• JOHANNISON, Ulf
**438 91 Landvetter (SE)**

(74) Representative: **Casalonga**
**Casalonga & Partners**
**Bayerstraße 71/73**
**80335 München (DE)**

(54) **WOUND CARE PRODUCT FOR TREATING CAVITY WOUNDS**

(57)     The present invention relates to a wound care product (in particular a wound dressing) for treating cavity wounds. The wound care product comprises a) an absorbent body that comprises absorbent fibers, wherein the absorbent body has a length-to-width ratio of at least 5, wherein the length is defined as the longest dimension of the absorbent body, and the width is defined as the longest dimension perpendicular to the length; and b) a porous cover surrounding at least a part of the absorbent body, wherein the porous cover has a length-to-width ratio of at least 5, wherein the length is defined as the longest dimension of the porous cover, and the width is defined as the longest dimension perpendicular to the length.

Fig. 1c

**(Cont. next page)**

(52) Cooperative Patent Classification (CPC): (Cont.)

C-Sets
**A61L 15/24, C08L 29/04**

**Description**

**FIELD OF THE INVENTION**

The present invention relates to a wound care product (in particular a wound dressing) for treating cavity wounds. The wound care product comprises

a) an absorbent body, wherein the absorbent body comprises absorbent fibers, wherein the absorbent body has a length-to-width ratio of at least 5, wherein the length is defined as the longest dimension of the absorbent body, and the width is defined as the longest dimension perpendicular to the length; and
b) a porous cover surrounding at least a part of the absorbent body.

**BACKGROUND OF THE INVENTION**

Cavitary wounds (also referred to as "cavity wounds") are wounds that have formed cavities. Timmons and Cooper ("How to systematically assess a patient with a cavity wound". Wounds UK 4(2): 4-10; 2008) define a cavity wound as one which extends beyond the layers of the dermis. Other authors suggest that a wound should be considered a cavity when there is a depth of 2 cm or more from the wound edge to the wound bed (Vowden K (2016) Defining, assessing and managing cavity wounds; Wounds UK 12(1): 18-23).

Cavity wounds that are deeper than 2 cm to the base of the wound are generally considered deep cavitary wounds (also referred to as "deep cavity wounds"). In deep cavity wounds, very often the underlying structures such as fascia, tendons, muscles and bone are exposed. Cavity wounds can result from a variety of traumatic incidents, surgical procedures, or chronic medical conditions, and present a complex set of challenges, including increased susceptibility to infection, compromised blood supply, potential impairment of underlying structures such as nerves and blood vessels, and the formation of chronic non-healing wounds.

The treatment of cavity wounds, in particular deep cavity wounds, presents a number of clinical and practical challenges for healthcare professionals. They often require debridement as well as biofilm, infection, and exudate management. In particular, cavity wounds are at risk of infection due to the presence of exudate, sloughy tissue and the potential for exudate to accommodate (or "pool") in the base of the wound, potentially leading to bacterial proliferation Appropriate exudate management, thus, is a key component of cavity wound management.

Exudates are a normal occurrence in the wound healing process, but they may cause problems when present in the wrong amount, in the wrong place or when of the wrong composition (World Union of Wound Healing Societies [WUWHS], 2019). Specifically, the wound bed ideally should be mildly moist to allow transport of nutrients, signaling molecules and immunological factors, as well as for proliferation and migration of the epithelial cells that initiate repair. Excess exudate delays wound healing because it typically contains high concentrations of inflammatory molecules.

While exudate release from the wound bed is due to a normal inflammatory response, aimed to increase the local vascular permeability so that immune cells can migrate to the wound, exudate accumulation, known as "pooling", is generally undesirable (Gefen A (2019) How medical engineering has changed our understanding of chronic wounds and future prospects. Med Eng Phys 72: 13-18; Lustig A, Alves P, Call E et al (2021) The sorptivity and durability of gelling fibre dressings tested in a simulated sacral pressure ulcer system. Int Wound J 18(2): 194-208). Excessive exudate is known to disturb healing and may cause cell and tissue damage, for example, by creating a conducive environment for pathogen growth or by wetting periwound skin, which results in maceration.

Preventing or at least minimizing exudate pooling and its negative impact on healing typically requires high absorption and retention of the dressing (including when subjected to mechanical forces), as well as intimate and continuous contact between the absorptive dressing surface and the wound bed. Providing for an intimate and continuous contact between the dressing surface and the wound bed, however, is challenging when it comes to treating cavity wounds.

Thus, one particular challenge encountered in the treatment of cavity wounds is ensuring an intimate and continuous contact between the dressing surface and the wound bed and providing for a beneficial moisture level, i.e. providing for a moist wound environment while avoiding or minimizing "pooling" of wound exudate in the wound cavity.

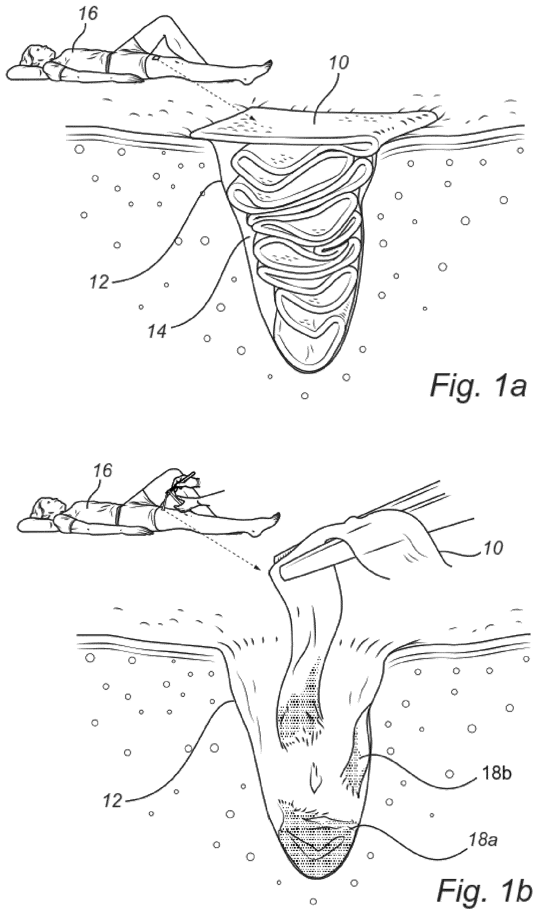

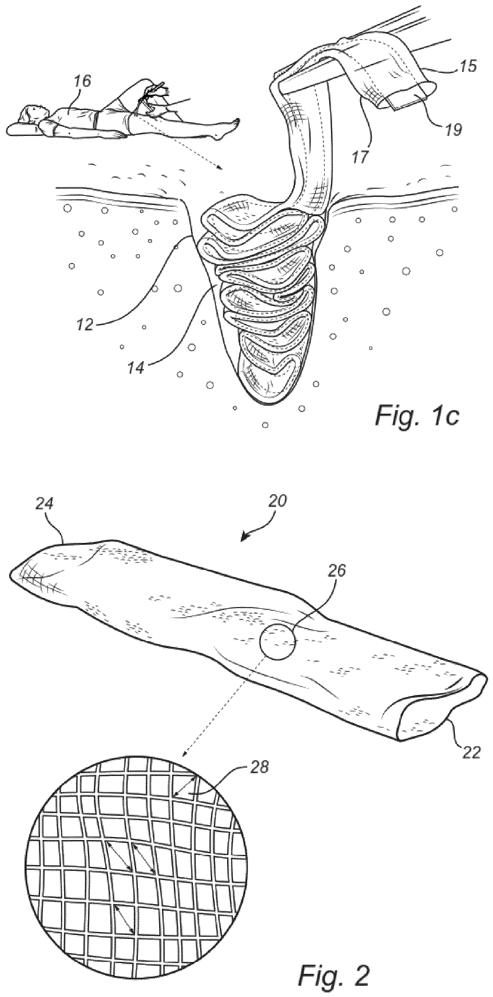

For treating cavity wounds, in particular deep cavity wounds, elongated wound dressings, such as so-called "ribbon dressings", have been proven useful as these wound dressings can be folded into the wound and, thus, provide for a contact between the dressing surface and the wound bed almost over the whole wound surface (see Fig. 1).

When such "elongated" wound dressings are made from or comprise an absorbent, hydrogel-forming material (often referred to as "gelling" wound dressings), a beneficial moist wound environment can be provided over a large area of the wound surface. Usually, after folding an elongated wound dressing into the wound cavity, the wound is covered with a secondary wound dressing, absorbent pad, gauze, or the like. An excess of wound exudate can then be transported from the wound via the elongated wound dressing (folded into the wound) into the secondary wound dressing, absorbent pad, gauze, or the like. In this way, excess wound exudate can be removed from the wound (i.e. pooling of wound exudate is reduced), and the intervals of changing the wound dressing can be prolonged.

There are, however, still several challenges associated with the treatment of cavity wounds, in particular deep cavity wounds.

One challenge is to provide for complete and easy removal of the wound dressing from the wound without leaving any pieces or fragments of the wound dressing behind. This is highly important because leaving behind pieces or fragments of the wound dressing in the wound would delay the wound healing process. The reason for potential difficulties associated with the removal of a "spent" dressing is that, upon absorption of wound exudate and formation of a hydrogel, the tensile strength of the dressing usually decreases. In addition, hydrogel-forming dressings swell, i.e. increase in volume, as they absorb wound exudate. As a result, the dressing may become "stuck" in the wound cavity and tear when being removed by pulling on its end. For addressing this problem, it has been proposed to stitch-bond (reinforce) the gelling material with a non-gelling strengthening yarn. The yarn shall reinforce the dressing to provide tensile strength even when the dressing is wet. A commercial product that applies such a stitch-bonding approach is the Aquacel® wound dressing.

However, while stitch-bonding a gelling dressing with a non-gelling yarn may reinforce the *overall* dressing, it may still happen that small fragments of the gelling material are ripped off during removal of the wound dressing and remain in the wound.

In view of the above, there is still a high demand for wound care products for treating cavity wounds, in particular deep cavity wounds. In particular, there is a desire for wound care products that provide for a moist environment (which helps facilitate autolytic debridement and wound healing) but avoid or minimize the pooling of wound exudate in the wound cavity. Further, it is desired that the wound care products can be removed from the wound without or with minimal disintegration upon removal.

## SUMMARY OF THE PRESENT INVENTION

It is an object of the present invention to overcome or minimize all or some of the above-mentioned problems.

In particular, based on the above, it is an object of the present invention to provide for a wound care product for treating cavity wounds that solves all or at least a sub-set of the above problems.

These and other objects are achieved by the wound care product of claim 1. In particular, by providing an absorbent body, the wound dressing can be adapted to provide a beneficial moist environment (which helps facilitate autolytic debridement and wound healing) to the wound but at the same time avoid a pooling of wound exudate in the wound cavity.

Further, by providing a porous cover that surrounds at least a part of the absorbent body, the wet tensile strength of the overall wound care product can be increased so that the wound care product, in particular the absorbent body, does not rupture upon pulling the wound care product out of a wound. By providing a *porous* cover, wound exudate can reach the absorbent body while, at the same time, the overall dressing is made more stable, in particular during use.

Further, by surrounding (at least a part of) the absorbent body with the porous cover (instead of using a stitch-bonding approach for reinforcing the absorbent body), it can be avoided that small fragments of the absorbent body are ripped off during removal of the wound care product. And even if small fragments of the absorbent body are ripped off during removal of the wound care product the same can be held back by (or, in other words: kept within) the porous cover.

The term "surrounding at least a part of the absorbent body", as used herein, is to be understood as follows:

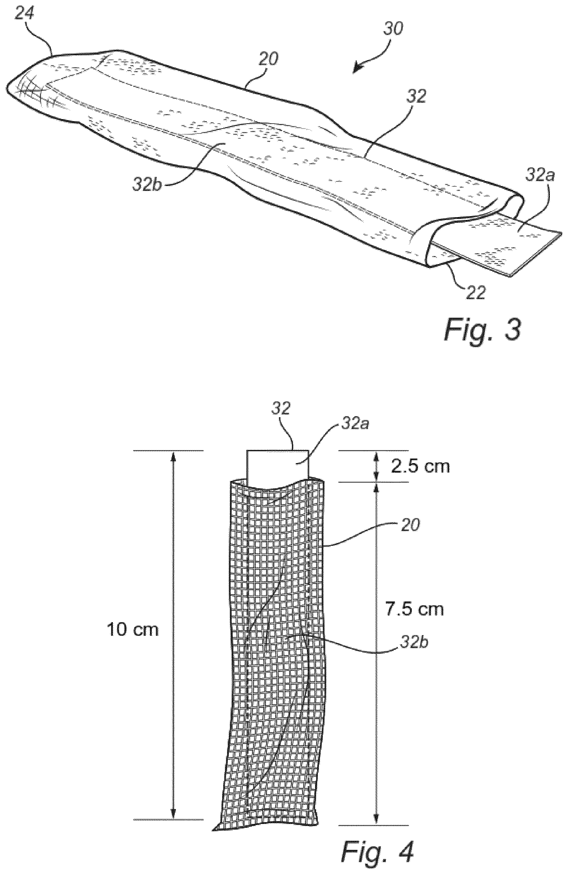

"Surrounding" is to be understood in the sense of surrounding around the entire circumference (like a sock surrounds the foot and part of the leg; or like a "Loferl" - a component of a traditional Bavarian costume - surrounds a part of the leg, more precisely the calf). Thus, that part of the absorbent body that is surrounded by the porous cover is surrounded around its entire circumference. For example, in the embodiment of Fig. 3, the part of the absorbent body that is provided within the porous cover is surrounded by the porous cover along its entire circumference; and that part that sticks out of the porous cover is not surrounded by the porous cover.

The reference to surrounding *at least a part* of the absorbent body (meaning that not the entire absorbent body must be surrounded by the porous cover) means that the absorbent body may stick out of the porous cover (as shown in Fig. 3). Or, in other words, surrounding *at least a part* may be understood in the sense of surrounding at least a part of the length of the absorbent body. In any case, as noted above, that part of the absorbent body that is surrounded by the porous cover must be surrounded around its entire circumference. From the above follows that a situation of one sheet simply lying on top of another sheet is not encompassed by the term "surrounding at least a part of the absorbent body". In view of the above, the feature that the porous cover surrounds at least a part of the absorbent body is to be interpreted as meaning that at least a part of the absorbent body is provided *within* the porous cover, or *within* an interior volume defined by the porous cover.

Moreover, by providing the absorbent body in a length-to-width ratio of at least 5 (wherein the length is defined as the longest dimension of the absorbent body, and the width is defined as the longest dimension perpendicular to the length), the wound care product is an elongated product (assuming that the contour of the porous cover follows the contour of the absorbent body), which allows to fold it into wound cavities, such that a large area of the wound surface is covered by the wound care product and the health care practitioner has great flexibility in adapting the wound dressing to different kinds of wounds.

In view of the above, the wound care product according to the present invention leads to at least the following advantages:

- It covers a large area of the wound cavity, i.e. it is suitable for treating cavity wounds, even deep cavity wounds.
- It provides for a beneficial moist environment but at the same time avoids or minimizes a pooling of wound exudate in the wound cavity.
- It avoids or minimizes a rupture of the wound care product upon pulling the wound care product out of a wound and avoids or minimizes that small fragments of the absorbent body are ripped off during removal of the wound care product.
- Even if small fragments of the absorbent body are ripped off during removal of the wound care product, the same can be held back by (kept within) the porous cover. Thus, the porous cover acts as a filter that can capture any loss fragments or pieces of the absorbent cover which may otherwise be left in the wound.

In a *first aspect,* the present invention relates to a wound care product according to claim 1.

The invention is described in more detail hereinbelow, with reference to the enclosed figures, which are only meant to be illustrative, wherein:

**Fig. 1a** illustrates the folding of a ribbon wound dressing in a wound cavity.

**Fig. 1b** illustrates the rupturing of a non-inventive ribbon wound dressing upon being pulled out of a wound cavity.

**Fig. 1c** illustrates how an exemplary wound care product, in the form of a wound dressing, according to an embodiment of the present invention, is pulled out of a cavity wound.

**Fig. 2** shows a schematic drawing of an exemplary porous cover that is open on one of its two ends and closed on the other one of its ends.

**Fig. 3** shows an exemplary embodiment of a wound care product according to the present invention, comprising an absorbent body having a ribbon structure and the porous cover of Fig. 2, wherein the absorbent body extends through the open end of the porous cover.

**Fig. 4** illustrates the determination of the length%.

**Fig. 5** shows an exemplary embodiment of a wound care product according to the present invention where the entire absorbent body is surrounded by the porous cover and the porous cover is closed on both of its ends.

**Fig. 6** shows an exemplary embodiment of a wound care product according to the present invention where the entire absorbent body is surrounded by the porous cover and the porous cover is closed on one of its two ends and open on the other one.

**Fig. 7** shows a photograph of an exemplary embodiment of a porous cover during the measurement of the wet tensile strength.

## DETAILED DESCRIPTION OF THE INVENTION

The present invention is at least partly based on the surprising finding that the above-mentioned drawbacks can be overcome by surrounding at least a part of an absorbent body with a porous cover.

In a *first aspect,* the present invention relates to a wound care product according to claim 1. The wound care product comprises an absorbent body that comprises absorbent fibers. The wound care product further comprises a porous cover that surrounds at least a part of the absorbent body. Further, the absorbent body has a length-to-width ratio of at least 5. The length is defined as the longest dimension of the absorbent body, and the width is defined as the longest dimension perpendicular to the length.

By having a length-to-width ratio of at least 5, the absorbent body may be described as an elongated structure. Such an absorbent body has two opposite ends.

According to a preferred embodiment, the porous cover surrounds at least one of the two opposite ends of the absorbent body and is closed around said one end (like a sock is closed at the toes). In this way, if the end of the absorbent body that is surrounded by the porous cover is introduced into the wound first, the absorbent body is necessarily removed from the wound together with the porous cover. For example, for removing the entire wound care product from the wound, one may pull on the porous cover, and the whole wound care product will follow. This situation is illustrated in Fig. 1c.

According to an embodiment, the entire wound care product of the present invention is an elongated structure, i.e. a structure that is significantly longer than it is wide. This elongated structure can be folded into a wound cavity and, thus, is suitable for treating cavity wounds.

The wound care product of the present invention is preferably a wound dressing.

As a person skilled in the art would understand, the term "absorbent body" implies that the absorbent body is adapted to absorb wound liquids.

The absorbent body preferably has a length-to-width ratio of at least 6, preferably at least 7, more preferably at least 8, more preferably at least 9, more preferably at least 10.

Preferably, the width of the absorbent body is greater than 0.5 cm, preferably greater than 0.6 cm, more preferably greater than 0.7 cm, more preferably greater than 0.8 cm, more preferably greater than 0.9 cm. It is also preferred that the width of the absorbent body is smaller than 5 cm, preferably smaller than 4 cm, more preferably smaller than 3 cm, more preferably smaller than 2.5 cm. It is particularly preferred that the width of the absorbent body is greater than 0.5 cm and smaller than 5 cm, preferably greater than 0.5 cm and smaller than 4 cm, more preferably greater than 0.5 cm and smaller than 3 cm, more preferably greater than 0.5 cm and smaller than 2.5 cm, more preferably greater than 0.6 cm and smaller than 2.5 cm, more preferably greater than 0.7 cm and smaller than 2.5 cm, more preferably greater than 0.8 cm and smaller than 2.5 cm.

The length of the absorbent body is preferably more than 5 cm, more preferably more than 10 cm, more preferably more than 15 cm, more preferably more than 20 cm, more preferably more than 25 cm, more preferably more than 30 cm, more preferably more than 35 cm, more preferably more than 40 cm.

According to an embodiment, the absorbent body has a ribbon structure. As a person skilled in the art of wound dressings knows, a ribbon structure is a generally sheet-like structure and may be described as having a first side and a second side, wherein said first side and said second side are opposite to each other and extend in the horizontal direction of the overall structure, and wherein said thickness relates to the distance, perpendicular to the horizontal direction, between the first surface and the second surface.

According to an embodiment, the wound care product, which is preferably a wound dressing, is a ribbon wound dressing. Ribbon wound dressings are suitable for treating cavity wounds and are also referred to in the art as "ribbon type wound dressings", "ribbon dressings", "ribbon cavity dressings", "ribbon type cavity dressings", "ribbon type cavity wounds dressings", and the like.

According to another embodiment, the absorbent body has a rope structure. As a person skilled in the art of wound dressings knows, a rope structure generally refers to a three-dimensional object characterized by a cylindrical shape. Also, a "rope structure", as referred to herein, does not need to have a perfectly round cross-sectional profile but also includes structures with a flattened geometry, i.e. structures having an oval cross-sectional profile.

According to an embodiment, the wound care product, preferably a wound dressing, is a rope wound dressing. Rope wound dressings are also suitable for treating cavity wounds and are also referred to in the art as "rope type wound dressings", "rope dressings", "rope cavity dressings", "rope type cavity dressings", "rope type cavity wounds dressings", and the like.

Accordingly, in embodiments of the invention the wound care product is a ribbon wound dressing or a rope wound dressing.

According to an embodiment, the absorbent body comprises the absorbent fibers in an amount of more than 80 wt.%, preferably more than 85 wt.%, more preferably more than 90 wt.%, more preferably more than 95 wt.%, based on the total weight of the absorbent body. In embodiments of the invention, the absorbent body consists of absorbent fibers. By providing a high percentage of absorbent fibers, optimal absorbency and the provision of a beneficial moisture level for the wound are ensured.

It is preferred that the absorbent fibers are gelling absorbent fibers. According to the IUPAC Gold Book (https://doi.org/10.1351/goldbook.G02600), a "gel" is a *"non-fluid colloidal network or polymer network that is expanded throughout its whole volume by a fluid"*. A "hydrogel" is a gel in which the swelling agent is water (https://doi.org/10.1351/goldbook.HT07519). Within the meaning of the present invention, the term "gelling absorbent fibers" should be understood to refer to fibers that form a hydrogel upon contact with an aqueous liquid.

Accordingly, it is preferred that the absorbent body is gelling, i.e. the absorbent body forms a hydrogel upon contact with aqueous liquids. In line with the above, by providing a high percentage of absorbent fibers, optimal absorbency and the provision of a beneficial moisture level for wound healing are ensured.

According to embodiments of the invention, the absorbent fibers comprise a member selected from the group consisting of polyacrylates, polyvinyl alcohol (PVA), preferably cross-linked PVA; polysaccharides, preferably modified cellulose, more preferably CMC; polyurethane polymer with polyethylene glycol and/or polypropylene functionalities; alginate; and copolymers thereof. The gelling absorbent fibers may also consist of a member selected from the group consisting of polyacrylates, polyvinyl alcohol (PVA), preferably cross-linked PVA; polysaccharides, preferably modified cellulose, more preferably CMC; polyurethane polymer with polyethylene glycol and/or polypropylene functionalities; alginate; and copolymers thereof.

According to embodiments of the invention, the gelling absorbent fibers comprise or consist of polyvinyl alcohol (PVA), preferably cross-linked PVA, CMC, or polyacrylate. For example, the gelling absorbent fibers may comprise or consist of cross-linked PVA. Cross-linked PVA has been shown to possess advantageous properties for applications in the treatment of cavity wounds, such as high absorbency, and the provision of a beneficial moisture level for the wound. Cross-linked PVA fibers are disclosed in US 2013/0323195 and US 2013/0274415, the content of which relating to fiber materials is hereby incorporated by reference.

According to an embodiment, the absorbent body is a substrate of non-woven fibers. For example, the absorbent body may be a substrate of non-ionic, non-woven fibers, preferably a web of non-ionic, non-woven fibers. In accordance with the present disclosure, and also in accordance with the universally accepted understanding of the skilled person, a "non-woven" is defined as sheet or web structures bonded together by entangling fiber or filaments mechanically, thermally, or chemically, but not (as is conventionally done for fabrics) by weaving or knitting. Non-wovens are defined by ISO standard 9092 and CEN EN 29092. Non-woven substrates or webs are typically flat, porous sheets that are made directly from separate fibers or from molten plastic or plastic films. A "non-ionic" fiber, in accordance with the present disclosure, describes a fiber that is substantially not ionizing in aqueous solution at a pH value of 7.

Without limitation, suitable *non-ionic fiber materials* include or are polyvinyl alcohol, polysaccharides, and polymers comprising polyethylene glycol (PEG) and/or polypropylene glycol, for example polyurethane with PEG functionalities, such as the polymer fibers disclosed in WO 2013/041620.

In some embodiments, the non-ionic fiber material is or comprises a polyvinyl alcohol, preferably wherein the polyvinyl alcohol is cross-linked. At least one polyvinyl alcohol *copolymer* may also be used. In the case of a polyvinyl alcohol copolymer, polyethylene vinyl alcohol may be used, for example.

In some embodiments the non-ionic fiber material comprises a plurality of fibers comprising polyvinyl alcohol, such as the plurality of fibers disclosed in US 2013/0323195 and/or US 2013/0274415, the content of which relating to fiber materials is hereby incorporated by reference.

In some embodiments, the non-ionic fiber material comprises a polyurethane polymer with polyethylene glycol (PEG) and/or polypropylene glycol functionalities, such as the polymer fibers disclosed in WO 2013/041620.

If polyvinyl alcohol copolymers are used as non-ionic fiber materials, the properties of the fibers may be adjusted in a targeted manner as appropriate by modifying the chemical structure of the polymer. For example, in the event of use of polyethylene vinyl alcohol, the number of OH groups may be reduced. However, other functional groups may also be introduced into the fibers by means of copolymerization. Thus, polyvinyl alcohol copolymers make further non-ionic fiber materials available.

Polyethylene vinyl alcohol, polyvinyl alcohol styrene, polyvinyl alcohol vinyl acetate, polyvinyl alcohol vinyl pyrrolidone, polyvinyl alcohol ethylene glycol and/or polyvinyl alcohol, particularly preferably polyethylene vinyl alcohol, polyvinyl alcohol vinyl acetate, polyvinyl alcohol vinyl pyrrolidone, polyvinyl alcohol vinylamine, polyvinyl alcohol acrylate, polyvinyl alcohol acrylamide, polyvinyl alcohol ethylene glycol and/or polyvinyl alcohol and particularly preferably polyvinyl alcohol may all be used as a non-ionic fiber material, in accordance with embodiments of the present invention. Block copolymers and/or graft copolymers and/or block and graft copolymers, statistical or alternating systems and any mixtures of these are used as non-ionic fiber material.

A polyvinyl alcohol copolymer may be used in unsubstituted or in partially substituted form. In the event of partial substitution, partial substitution of the OH groups by -O(C=O)-R or -OR is included, wherein R, in each case independently of one another, stands for a C1-C4 alkyl group. In this case a C1-C4 alkyl group is understood to be methyl, ethyl, propyl, iso-propyl, 1-butyl, 2-butyl, or tert-butyl.

Furthermore, the non-ionic fiber material may be formed as a polymer blend. In this case a polymer blend is understood to be a physical mixture of at least two polymers from the melt or from the solution.

In order to form such a polymer blend, *further polymers* may be used, such as for example alginates, cellulose ethers, such as carboxymethyl celluloses, methyl celluloses, ethyl celluloses, hydroxymethyl celluloses, hydroxyethyl celluloses, hydroxyalkyl methylcelluloses, hydroxypropyl celluloses, cellulose esters, such as cellulose acetate, oxidised celluloses, bacterial celluloses, cellulose carbonates, gelatines, collagens, starches, hyaluronic acids, pectins, agar, polyacrylates, polyvinyl amines, polyvinyl acetates, polyethylene glycols, polyethylene oxides, polyvinyl pyrrolidones, polyurethanes or nongelling further polymers, such as for example polyolefins, celluloses, cellulose derivatives, regenerated celluloses such viscoses, polyamides, polyacrylonitriles, polyvinyl chlorides, chitosans, polylactides, polyglycolides, polyester amides, polycaprolactones, polyhexamethylene terephthalates, polyhydroxybutyrates, polyhydroxyvalerates or polyesters.

In some embodiments, the non-ionic fiber material comprises a polymer that is cross-linked, in particular cross-linked polyvinyl alcohol. The term *"cross-linked"* is used herein to describe a material comprising a plurality of polymer molecules which are interlinked by a chemical bond, in particular a covalent bond or an ionic bond, or by a physical cross-link, such as in thermoplastic elastomers.

In some embodiments, the non-ionic fiber material is cross-linked by heat or chemical treatment, in particular by heat. Cross-linked non-ionic fiber materials are capable of forming a swollen coherent gel upon absorbing a liquid.

In embodiments of the present invention, the average diameter of the fibers of the absorbent body is preferably in the range of 50 nm to 1000 $\mu$m, preferably 1 $\mu$m to 100 $\mu$m, further preferably from 5 - 25 $\mu$m.

According to an embodiment, the absorbent body comprises a polyacrylate polymer. According to another embodiment, the absorbent body does not comprise a polyacrylate polymer. According to another embodiment, the absorbent body does not comprise a polyacrylate polymer in fiber form.

It is possible to attach (for example by ultrasonic welding, gluing, or the like) the absorbent body to the porous cover. Such an attachment avoids a movement of the absorbent body inside the porous cover. For example, by attaching the absorbent body to the porous cover, it can be avoided that the absorbent body folds over itself within the porous cover. If the absorbent body is attached to the porous cover, it is preferred that the attachment is realized by means of attachment points, for example by means of ultrasonic spot welding, spot gluing, or the like. In another embodiment, the absorbent body is not attached to the porous cover.

According to one embodiment, the absorbent body has an area "A" and the porous cover defines an internal area "B", wherein "A" is at most 90% of "B". In this way, the porous cover provides for additional space to compensate for a swelling of the absorbent core upon absorption of wound exudate.

In embodiments of the invention, the absorbent body has a free swell absorptive capacity of at least 5 times its own weight, as measured by EN 13726:2023-12, preferably at least 10 times, more preferably at least 15 times, more preferably at least 20 times, more preferably at least 25 times.

It is also preferred that the absorbent body has a fluid retention capacity (as determined according to EN 13726:2023-12) of at least 60%, preferably at least 70%, more preferably at least 80%.

In embodiments of the invention, the absorbent body does not comprise non-gelling fibers.

Moreover, in embodiments of the invention, the absorbent body is not stitch-bonded with a non-gelling yarn.

The absorbent body may also comprise additives. For example, the absorbent body may comprise an antimicrobial agent. In embodiments of the invention, the antimicrobial agent comprises silver. In embodiments of the invention, the silver is metallic silver. In embodiments of the invention, the silver is a silver salt. In embodiments of the invention, the silver salt is silver sulfate, silver chloride, silver nitrate, silver sulfadiazine, silver carbonate, silver phosphate, silver lactate, silver bromide, silver acetate, silver citrate, silver CMC, silver oxide. In embodiments of the invention, the silver salt is silver sulfate. In embodiments of the invention, the antimicrobial agent comprises a monoguanide or biguanide. In embodiments of the invention, the monoguanide or biguanide is chlorhexidine digluconate, chlorhexidine diacetate, chlorhexidine dihydrochloride, polyhexamethylene biguanide (PHMB) or a salt thereof, or polyhexamethylene monoguanide (PHMG) or a salt thereof. In embodiments of the invention, the biguanide is PHMB or a salt thereof. In embodiments of the invention, the antimicrobial agent comprises a quaternary ammonium compound. In embodiments of the invention, the quaternary ammonium compound is cetylpyridinium chloride, benzethonium chloride, or poly-DADMAC. In embodiments of the invention, the antimicrobial agent comprises triclosan, sodium hypochlorite, copper, hydrogen peroxide, xylitol, or honey.

In embodiments of the invention, the fiber material further comprises an antimicrobial coating that comprises an antimicrobial agent and one or more polymers, wherein the one or more polymers are selected from the group consisting of cellulosic polymers, neutral poly(meth)acrylate esters, polyvinylpyrrolidone, polyvinylpolypyrrolidone, and combinations thereof.

In embodiments of the invention, the one or more polymers in the antimicrobial coating are cellulosic polymers, preferably selected from the group consisting of hydroxypropylmethylcellulose (HPMC), hydroxypropylcellulose (HPC), methylcellulose (MC), and ethylcellulose (EC), preferably hydroxypropylcellulose (HPC). In embodiments of the invention, the one or more polymers in the antimicrobial coating is hydroxypropylcellulose (HPC).

In embodiments of the invention, the antimicrobial agent in the antimicrobial coating is or comprises silver, preferably silver oxide or a silver salt.

In embodiments of the invention, the wet tensile strength of the porous cover is higher than the wet tensile strength of the absorbent body. The wet tensile strength is determined as set out below. By using a porous cover that has a higher wet tensile strength than the absorbent body, the porous cover reinforces the absorbent body such that the same is less likely to tear apart upon being pulled out of a cavity wound. This significantly minimizes the risk of leaving any pieces or fragments of the wound dressing behind in the wound cavity. As noted above, leaving behind pieces or fragments of the wound dressing behind in the wound cavity should be avoided as the same may delay wound healing.

Preferably, in embodiments of the invention, the wet tensile strength of the porous cover is at least 1.5 times the wet tensile strength of the absorbent body. In embodiments, the wet tensile strength of the porous cover is at least 2 times the wet tensile strength of the absorbent body. In embodiments, the wet tensile strength of the porous cover is at least 3 times the wet tensile strength of the absorbent body. In embodiments, the wet tensile strength of the porous cover is at least 4 times the wet tensile strength of the absorbent body. In embodiments, the wet tensile strength of the porous cover is at least 5 times the wet tensile strength of the absorbent body. In embodiments, the wet tensile strength of the porous cover is at least 6 times the wet tensile strength of the absorbent body. In embodiments, the wet tensile strength of the porous cover is at least 7 times the wet tensile strength of the absorbent body. In embodiments, the wet tensile strength of the porous cover is at least 8 times the wet tensile strength of the absorbent body. In embodiments, the wet tensile strength of the porous cover is at least 9 times the wet tensile strength of the absorbent body. In embodiments, the wet tensile strength of the porous cover is at least 10 times the wet tensile strength of the absorbent body.

In embodiments, the wet tensile strength of the absorbent body is between 1 and 10 N/20 mm, such as between 2 and 9 N/20 mm, or between 3 and 8 N/20 mm, such as between 3 and 7 N/20 mm, or between 4 and 6 N/20 mm, wherein the wet tensile strength is determined as set out below.

In embodiments, the wet tensile strength of the porous cover is greater than 20 N/20 mm, such as greater than 25 N/20 mm, or greater than 30 N/20 mm, or than 35 N/20 mm, or greater than 40 N/20 mm, or than 45 N/20 mm, or than 50 N/20 mm, or greater than 55 N/20 mm, or greater than 60 N/20 mm, or greater than 65 N/20 mm, or greater than 70 N/20 mm, or greater than 75 N/20 mm, or greater than 80 N/20 mm. Again, the wet tensile strength is determined as set out below. It has been found that if the wet tensile strength is in the specified ranges, the porous cover reinforces the absorbent body particularly well such that it is highly unlikely that the absorbent body tears apart upon being pulled out of a cavity wound.

The term "wet tensile strength" is to be understood as the maximum tensile force per unit width, as measured in accordance with EN 29073-3:1992 (with amendments as specified below), that a test piece will stand, in a wet state having absorbed a maximum amount of Solution A according to the "Free swell absorptive capacity method" EN 13726:2023-12 (with an absorption time of 10±1 minutes at 23 ±2 °C), before it breaks apart in said tensile strength test.

The method of measuring the "wet tensile strength", according to the invention, is modified vis-à-vis EN 29073-3:1992 in the following manner: i) the material is soaked according to EN 13726:2023-12 (with an absorption time of 10±1 minutes at 23 ±2 °C and before cutting the test piece); ii) the test piece had width of 20 mm; iii) a gauge length of 50 mm (i.e. distance between jaws according to section 7.2 of EN 29073-3:1992) is used; and iv) method is performed at a temperature of 23 ±2 °C and 50±5%rh. These deviations are all allowable alterations of EN 29073-3:1992 (see, e.g., notes in sections 6 and 7 of EN 29073-3:1992).

Solution A, as defined in EN 13726:2023-12, consists of a sodium chloride and calcium chloride solution containing 142 mmol of sodium ions and 2.5 mmol of calcium ions as the chloride salts. This solution has an ionic composition comparable to human serum or wound exudate. Said solution is prepared by dissolving 8.298 g of sodium chloride and 0.368 g of calcium chloride dihydrate in deionized water up to the "1 L" marking in a volumetric flask.

Further, it is preferred that the free swell absorptive capacity of the absorbent body is higher than the free swell absorptive capacity of the porous cover, wherein the free swell absorptive capacity is determined according to EN 13726:2023-12. In embodiments, the free swell absorptive capacity of the absorbent body is at least 50% greater than the free swell absorptive capacity of the porous cover. In embodiments, the free swell absorptive capacity of the absorbent body is at least 80% greater, such as at least 100% greater, than the free swell absorptive capacity of the porous cover. In embodiments, the free swell absorptive capacity of the absorbent body is at least 200% greater, such as at least 500% greater, than the free swell absorptive capacity of the porous cover. It is to be understood that when comparing the free swell absorptive capacity of the absorbent body and the porous cover, the comparison is made for equal weights of the absorbent body and the porous cover.

Further, it is preferred that the fluid retention capacity of the absorbent body is greater than the fluid retention capacity of the porous cover, wherein the fluid retention capacity is determined according to EN 13726:2023-12. When comparing the fluid retention capacity of the absorbent body and the porous cover, the fluid retention capacity expressed as "percentage of fluid retained" is meant. In embodiments, the fluid retention capacity (expressed as "percentage of fluid retained") of the absorbent body is at least 1.5 times the fluid retention capacity of the porous cover. This means that if the porous cover has a fluid retention capacity of 20%, the absorbent body has a fluid retention capacity of at least 30%. In embodiments, the fluid retention capacity of the absorbent body is at least 1.8 times, such as at least 2 times, the fluid retention capacity of the porous cover. In embodiments, the fluid retention capacity of the absorbent body is at least 3 times, such as 4 or 5 times, the fluid retention capacity of the porous cover.

By providing an absorbent body that has a higher free swell absorptive capacity and/or fluid retention capacity than the porous cover, wound liquid will be transported from the wound, through the pores of the porous body, into the absorbent body where it is then stored. In this way, an advantageous moisture level for wound healing can be realized.

In embodiments, the porous cover is an elongated structure.

In embodiments, the porous cover has a length-to-width ratio of at least 5, wherein the length is defined as the longest dimension of said porous cover, and the width is defined as the longest dimension perpendicular to said length. In embodiments, the porous cover has a length-to-width ratio of at least 6, for example at least 7, or at least 8, or at least 9, or at least 10.

In embodiments, both the absorbent body and the porous cover have a length-to-width ratio of at least 6, such as at least 7, or at least 8, or at least 9, or at least 10.

It is particularly preferred that the contour of the porous cover follows the contour of the absorbent body.

As noted above, it is preferred that the entire wound care product, preferably a wound dressing, is a ribbon dressing or a rope dressing. Ribbon dressings and rope dressings are suitable for treating cavity wounds as they can be folded into the wound cavity (see Fig. 1).

Further, in all embodiments, it is preferred that the width of the porous cover is greater than the width of the absorbent body. In this way, the absorbent body can be introduced inside the porous cover, i.e. be surrounded by the porous cover, in a spread-out geometry, i.e. without being bent or folded over itself. In contrast, if the width of the porous cover would be smaller than the width of the absorbent body, the absorbent body would have to be bent or folded over itself for being introduced inside the porous cover, and a swelling of the absorbent body within the porous cover may be impaired.

In embodiments, the width of the absorbent body is not more than 90% of the width of the porous cover. In this way, there is sufficient volume provided for the absorbent body to swell within the porous cover.

In embodiments, the width of the porous cover is greater than 0.5 cm, preferably greater than 0.6 cm, more preferably greater than 0.7 cm, more preferably greater than 0.8 cm, more preferably greater than 0.9 cm.

It is also preferred that the width of the porous cover is smaller than 5 cm, preferably smaller than 4 cm, more preferably smaller than 3 cm, more preferably smaller than 2.5 cm.

According to an embodiment, the width of the porous cover is greater than 0.5 cm and smaller than 5 cm, such as greater than 0.5 cm and smaller than 4 cm, or greater than 0.5 cm and smaller than 3 cm, or greater than 0.5 cm and smaller than 2.5 cm, or greater than 0.6 cm and smaller than 2.5 cm, or greater than 0.7 cm and smaller than 2.5 cm, or greater than 0.8 cm and smaller than 2.5 cm.

In embodiments, the length of the porous cover is more than 5 cm, such as more than 10 cm, or more than 15 cm, or more than 20 cm, or more than 25 cm, or more than 30 cm, or more than 35 cm, or more than 40 cm.

In one embodiment, the length of the porous cover is greater than the length of the absorbent body.

In one embodiment, the porous cover is a tubular structure having two opposite ends. Within the meaning of the present invention, a "tubular structure" refers to a three-dimensional object characterized by a hollow, cylindrical shape. The distance between the two opposite ends defines the length of the porous cover. A "tubular structure", as referred to herein, does not need to have a perfectly round cross-sectional profile but also includes structures with a flattened geometry, i.e. structures having an oval cross-sectional profile. This makes particular sense considering that the porous cover is usually a textile material which shape varies depending on the external stimuli and when lying flat on the ground, the porous cover will likely collapse on itself.

In one embodiment, the porous cover is open on one of its two ends and closed on the other one. Such a structure is schematized in Fig. 2. In this embodiment, the absorbent body may extend through the opened end, such that a portion of the absorbent body is provided within the porous cover and another portion of the absorbent body is provided outside the porous cover. In this configuration, a certain percentage of the absorbent body (corresponding to the portion outside the porous cover) is not surrounded by the porous cover. Such a configuration is illustrated in Fig. 3. If the wound cavity is finally covered with an absorbent pad, wound dressing, gauze, or the like, a direct contact between the that part of the absorbent body that is located outside the porous cover and the absorbent pad, wound dressing, gauze, or the like can be achieved. This allows for a fast and unimpeded transport of wound exudate from the absorbent body into the absorbent pad, wound dressing, gauze, or the like, where the exudate can then be stored. In this way, the absorbent capacity is increased and the intervals for changing the wound dressing can be prolonged.

In view of structural integrity, wet tensile strength, and prevention of ripping off small fragments of the absorbent body upon removal of the wound care product from the wound, it is preferred that the majority of the absorbent body is surrounded by (i.e. provided within) the porous cover. In particular, it is preferred that at least 70 length% of the absorbent body, preferably at least 80 length%, more preferably at least 90 length%, more preferably at least 95 length%, are surrounded by the porous cover. "length%" is calculated as follows:

length% = (length of absorbent body surrounded by porous cover / total length of absorbent body) * 100%.

For example, if the absorbent body has a total length of 10 cm and 7.5 cm of the absorbent body are located within the porous cover (i.e. are surrounded by the porous cover) and 2.5 cm of the absorbent body are located outside the porous cover, 75 length% of the absorbent body are surrounded by the porous cover. This situation is illustrated in Fig. 4.

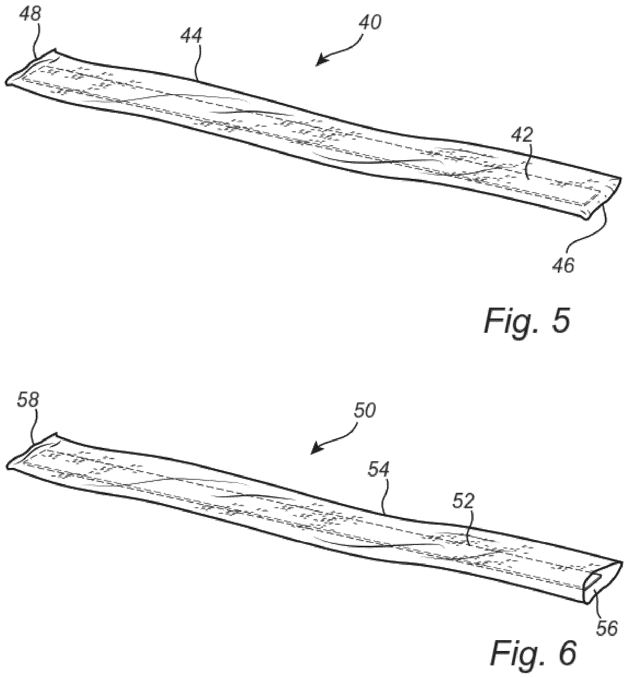

Alternatively, the entire absorbent body (i.e. the full length of the absorbent body, i.e. 100 length%) can be surrounded by the porous cover. That means that the entire absorbent body is provided within the porous cover and no parts of the absorbent body extend outside the porous cover. In this case, the porous cover can be closed on both of its two ends (Fig. 5), or can be open on one of its ends (Fig. 6). This is advantageous in terms of easy handling as the practitioner does not need to pay attention which end of the wound care product must be introduced into the wound cavity first or where to pull when removing the wound care product from the wound. Further, this is advantageous in terms of structural integrity and robustness of the wound care product because the absorbent body is reinforced by the porous cover along its full length.

According to one embodiment, both ends of the porous cover are closed.

Further, in embodiments, the wound care product comprises only one porous cover.

Further, in embodiments, the porous cover is non-gelling. That means that it does not form a hydrogel upon contact with an aqueous liquid.

In one embodiment, the absorbent body is more hydrophilic than the porous cover. When a hydrophilic surface contacts a water droplet, the water droplet is spread out or absorbed. In contrast, hydrophobic surfaces typically cause water to bead up or to form droplets rather than spreading or being absorbed. The "hydrophilicity" or "hydrophobicity" can be determined based on the contact angle determined according to ASTM D7334-08 (Reapproved 2022).

In one embodiment, the porous cover is made of a material that defines a contact angle of at least 80 degrees, wherein the contact angle is determined according to ASTM D7334-08. In another embodiment, the porous cover is made of a material that defines a contact angle of at least 90 degrees wherein the contact angle is determined according to ASTM D7334-08. In another embodiment, the porous cover is made of a material that defines a contact angle of at least 100 degrees, wherein the contact angle is determined according to ASTM D7334-08. In another embodiment, the porous cover is made of a material that defines a contact angle of at least 110 degrees, wherein the contact angle is determined according to ASTM D7334-08.

The reference to the porous cover being "made of a material that defines a contact angle of at least ..." is made, because due to the porous structure of cover, it is often not possible to perform contact angle measurements directly on the porous cover. Thus, the contact angles refer to the contact angles of a water droplet on a continuous surface that is made of the same material as the porous cover.

In embodiments, the porous cover comprises or consists of a member selected from the list consisting of nylon; viscose; polyester such as polyethylene terephthalate (PET), polybutylene terephthalate (PBT), and polytrimethylene terephthalate (PTT); polyethylene; polypropylene; acrylic fibers; olefin fibers; polytetrafluoroethylene-coated materials; silicone-coated materials, preferably silicone-coated viscose; materials coated with polyvinylchloride or with other vinyl-based materials; and polyamide. The inventors of the present invention have found that these materials are particularly well suitable for preventing an ingrowth into the absorbent body while allowing a fluid transfer to the absorbent body.

In embodiments, the porous cover is made of a fibrous material. The fibrous material is preferably yarn. The yarn may be a thread. As one of skill in the art knows, yarn is a spun agglomeration of fibers used for knitting, weaving or sewing. Thread usually consists of multiple yarns plied together, producing a long, thin strand used in sewing or weaving.

In embodiments, the porous cover is a knitted structure, a woven structure, a welded structure, or a non-woven structure.

In embodiments, the average size of the pores of the porous cover (also referred to herein as average "pore size") is smaller than the smallest dimension of the absorbent body. As referred to herein, the "pore size" generally refers to the largest dimension of the pores. If the porous cover is a mesh structure, for example a knitted or woven structure, the pore size is defined as the largest dimension of the pores in a lay-flat geometry. "Lay-flat geometry" refers to a situation where no external forces (except for gravitation) are applied onto the porous cover. The pore size can be measured by microscopy. The zoomed area of Fig. 2 shows a variety of pores, wherein the largest dimension (i.e. the pore size) is indicated for a few pores by a double arrow ( ↔ ).

In embodiments, the pores have an average pore size of 5 mm or less. In other embodiments, the pores have an average pore size of 4 mm or less. In other embodiments, the pores have an average pore size of 3 mm or less. In other embodiments, the pores have an average pore size of 2,5 mm or less. In other embodiments, the pores have an average pore size of 2 mm or less.

In embodiments, the pores have an average pore size of 0.15 mm to 5 mm, preferably 0.17 mm to 4 mm, more preferably 0.17 mm to 3 mm, more preferably 0.3 mm to 3 mm, more preferably 0.3 mm to 2.5 mm, more preferably 0.5 mm to 2.5 mm, more preferably 1 mm to 2 mm.

In embodiments, the pores have a homogenous pore size distribution. Preferably, the pore size of the largest pore is at most 1.5 times the pore size of the smallest pore.

It has been found that pores of the above-defines pore sizes are big enough to allow for a practically unhindered transport of wound exudate through the porous cover to the absorbent body over time and are small enough to inhibit a loss of potential fragments of the absorbent body into the wound. Or, in other words, even if small fragments of the absorbent body are ripped off (for example during removal of the wound dressing), such fragments are held back by the porous cover. The porous cover, thus, functions as a kind of fishing net or filter that prevents any potential fragments of the absorbent body from entering into the wound cavity. If the pores are too small, they might get blocked over time by components of the wound exudate, such as cells, proteins, and other solid materials.

In embodiments, the porous cover has a uniform material composition, wherein uniform material composition means that the material properties of the porous cover do not vary in terms of composition and vary not by more than 5%, preferably not by more than 1%, in terms of physical parameters characterizing the material across the entire area of the porous cover. This allows for an easy and cost-saving production of the porous cover. For example, if the porous structure is a knitted structure, it can be knitted from a single yarn or thread.

According to an embodiment, the width of the porous cover is greater than the width of the absorbent body. Preferably, the width of the porous cover is greater than the width of the absorbent body and the length of the porous cover is greater than the length of the absorbent body.

Furthermore, it has surprisingly been found that the re-wetting capability of the wound care product, comprising the porous cover surrounding the absorbent body, is not significantly different from the re-wetting capability of the absorbent body alone. The rewetting capability of a wound dressing refers to its ability to manage moisture in a wound by absorbing exudate while also preventing the wound from drying out. Maintaining an optimal moisture balance is crucial for the healing process. Also, it has been found that the re-wetting capability can be fine-tuned by modifying the material, in particular the hydrophobicity, of the porous cover. In terms of good wound healing, it is preferred that the wound care product has a re-wetting capability of 0.1 g or more, preferably 0.2 g or more, as determined by the re-wetting test described in the examples section.

According to an embodiment, the wound care product consists of the absorbent core and the porous cover.

## DETAILED DESCRIPTION OF THE DRAWINGS

Fig. 1a shows the folding of a prior art ribbon wound dressing 10 in a wound cavity 12 of a patient 16. Upon folding the wound dressing 10 into the wound cavity 12, a large part of the wound surface 14 is in contact with and covered by the wound dressing 10.

Fig. 1b illustrates the rupturing of a prior art ribbon wound dressing 10 upon being pulled out of a wound cavity 12 of a patient 16. Upon rupture of the wound dressing 10, small fragments 18a, 18b are left behind in the wound cavity 12.

Fig. 1c illustrates how a wound dressing 15 according to an example embodiment of the present invention is pulled out of a wound cavity 12.

Fig. 2 shows a schematic drawing of an example embodiment of a porous cover 20 that is open on one of its two ends 22 and closed on the other one of its ends 24. The zoomed area 26 illustrates a mesh structure of the porous cover 20 with a multitude of pores 28. The double arrows indicate the largest dimension (i.e. the pore size) of a few pores.

Fig. 3 shows an example embodiment of a wound care product 30 according to the present invention. The wound care product 30 comprises an absorbent body 32 having a ribbon structure and the porous cover 20 illustrated in Fig. 2. The porous cover 20 surrounds a part of the absorbent body 32. The absorbent body 32 extends through the open end 22, such that a portion 32b of the absorbent body 32 is provided within the porous cover 20 (i.e. is surrounded by the porous cover 20) and another portion 32a of the absorbent body 32 is provided outside the porous cover 20 (i.e. is not surrounded by the porous cover 20). The portion 32a outside the porous cover 20 can, for example, be used for efficiently transporting wound fluid to a secondary wound dressing.

Fig. 4 illustrates the determination of the length%. Fig. 4 is not drawn to scale. The absorbent body 32 has a total length of 10 cm. 7.5 cm of the absorbent body 32 are located within the porous cover 20 (i.e. are surrounded by the porous cover 20) and 2.5 cm of the absorbent body 32 are located outside the porous cover 20. This calculates to 75 length% of the absorbent body 32 being surrounded by the porous cover 20.

Fig. 5 shows an example embodiment of a wound care product 40 according to the present invention. In this embodiment, the entire absorbent body 42 is surrounded by the porous cover 44. The porous cover 44 is closed on both of its ends 46, 48.

Fig. 6 shows another example embodiment of a wound care product 50 according to the present invention. In this embodiment, the entire absorbent body 52 is surrounded by the porous cover 54. The porous cover 54 is closed on one of its ends 58 and open on the other one of its ends 56.

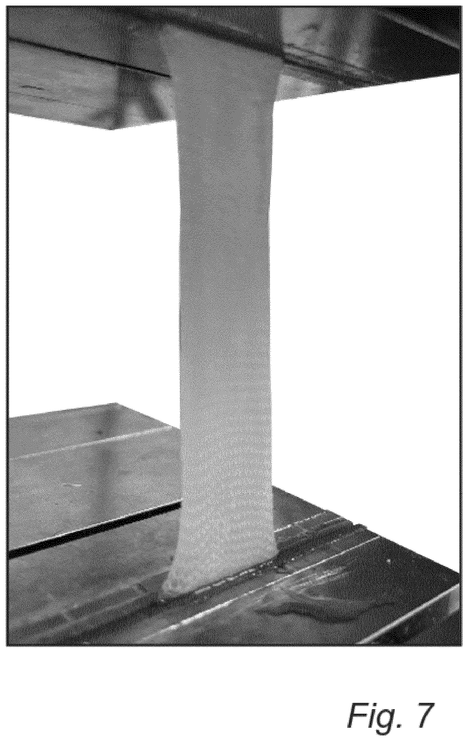

Fig. 7 shows an embodiment of a porous cover during the measurement of the wet tensile strength.

The present invention is also described by way of the following numbered Items, which may be combined with each other and/or with the embodiments of the description:

Item 1. Wound care product, comprising:

a) an absorbent body,

wherein said absorbent body comprises absorbent fibers,

wherein said absorbent body has a length-to-width ratio of at least 5, wherein the length is defined as the longest dimension of said absorbent body, and the width is defined as the longest dimension perpendicular to said length; and

b) a porous cover surrounding at least a part of said absorbent body.

Item 2. The wound care product of Item 1, characterized in that it is a wound dressing, preferably a ribbon dressing or a rope dressing.

Item 3. The wound care product of Item 1 or 2, wherein the absorbent body has a length-to-width ratio of at least 6, preferably at least 7, more preferably at least 8, more preferably at least 9, more preferably at least 10.

Item 4. The wound care product of any one of the preceding Items, wherein the width of the absorbent body is greater than 0.5 cm, preferably greater than 0.6 cm, more preferably greater than 0.7 cm, more preferably greater than 0.8 cm, more preferably greater than 0.9 cm.

Item 5. The wound care product of any one of the preceding Items, wherein the width of the absorbent body is smaller than 5 cm, preferably smaller than 4 cm, more preferably smaller than 3 cm, more preferably smaller than 2.5 cm.

Item 6. The wound care product of any one of the preceding Items, wherein the width of the absorbent body is greater than 0.5 cm and smaller than 5 cm, preferably greater than 0.5 cm and smaller than 4 cm, more preferably greater than 0.5 cm and smaller than 3 cm, more preferably greater than 0.5 cm and smaller than 2.5 cm, more preferably greater than 0.6 cm and smaller than 2.5 cm, more preferably greater than 0.7 cm and smaller than 2.5 cm, more preferably greater than 0.8 cm and smaller than 2.5 cm.

Item 7. The wound care product of any one of the preceding Items, wherein the absorbent body has a length of more than 5 cm, preferably more than 10 cm, more preferably more than 15 cm, more preferably more than 20 cm, more preferably more than 25 cm, more preferably more than 30 cm, more preferably more than 35 cm, more preferably more than 40 cm.

Item 8. The wound care product of any one of the preceding Items, wherein the absorbent body has a ribbon structure.

Item 9. The wound care product of any one of Items 1-7, wherein the absorbent body has a rope structure.

Item 10. The wound care product of any one of the preceding Items, wherein the absorbent body comprises the absorbent fibers in an amount of more than 80 wt.%, preferably more than 85 wt.%, more preferably more than 90 wt.%, more preferably more than 95 wt.%, based on the total weight of the absorbent body.

Item 11. The wound care product of Item 10, wherein the absorbent body consists of the absorbent fibers.

Item 12. The wound care product of any one of the preceding Items, wherein the absorbent fibers are gelling absorbent fibers, i.e. form a hydrogel upon contact with aqueous liquids.

Item 13. The wound care product of any one of the preceding Items, wherein the absorbent body is a gelling absorbent body, i.e. forms a hydrogel upon contact with aqueous liquids.

Item 14. The wound care of any one of the preceding Items, wherein the absorbent fibers comprise or consist of a member selected from the group consisting of polyacrylates, polyvinyl alcohol (PVA), preferably cross-linked PVA; polysaccharides, preferably modified cellulose, more preferably carboxymethyl cellulose (CMC); polyurethane polymer with polyethylene glycol and/or polypropylene functionalities; alginate; and copolymers thereof.

Item 15. The wound care product of Item 14, wherein the gelling absorbent fibers comprise or consist of polyvinyl alcohol (PVA), preferably cross-linked PVA, CMC, or polyacrylate, most preferably PVA.

Item 16. The wound care product of any one of the preceding Items, wherein the absorbent body is a substrate of non-woven fibers.

Item 17. The wound care product of any one of the preceding Items, wherein the absorbent body does not comprise polyacrylate polymer.

Item 18. The wound care product of any one of the preceding Items, wherein the absorbent body does not comprise polyacrylate polymer in fiber form.

Item 19. The wound care product of any one of the preceding Items, wherein the absorbent body has an area "A" and the porous cover defines an internal area "B", wherein "A" is at most 90% of "B".

Item 20. The wound care product of any one of the preceding Items, wherein the absorbent body has a free swell absorptive capacity of at least 5 times its own weight, as measured by EN 13726-12023-12, preferably at least 10 times, more preferably at least 15 times, more preferably at least 20 times, more preferably at least 25 times.

Item 21. The wound care product of any one of the preceding Items, wherein the absorbent body has a fluid retention capacity of at least 60%, preferably at least 70%, more preferably at least 80%, as determined according to EN 13726:2023-12.

Item 22. The wound care product of any one of the preceding Items, wherein the absorbent body does not comprise non-gelling fibers.

Item 23. The wound care product of any one of the preceding Items, wherein the wet tensile strength of the porous cover is higher than the wet tensile strength of the absorbent body, wherein the wet tensile strength is determined as described in the description.

Item 24. The wound care product of any one of the preceding Items, wherein the wet tensile strength of the porous cover is at least 1.5 times the wet tensile strength of the absorbent body, preferably at least 2 times, more preferably at least 3 times, more preferably at least 4 times, more preferably at least 5 times, more preferably at least 6 times, more preferably at least 7 times, more preferably at least 8 times, more preferably at least 9 times, more preferably at least 10 times.

Item 25. The wound care product of any one of the preceding Items, wherein the wet tensile strength of the absorbent body is between 1 and 10 N/20 mm, preferably between 2 and 9 N/20 mm, more preferably between 3 and 8 N/20 mm, more preferably between 3 and 7 N/20 mm, more preferably between 4 and 6 N/20 mm.

Item 26. The wound care product of any one of the preceding Items, wherein the wet tensile strength of the porous cover is greater than 20 N/20 mm, preferably greater than 25 N/20 mm, more preferably greater than 30 N/20 mm, more preferably greater than 35 N/20 mm, more preferably greater than 40 N/20 mm, more preferably greater than 45 N/20 mm, more preferably greater than 50 N/20 mm, more preferably greater than 55 N/20 mm, more preferably greater than 60 N/20 mm, more preferably greater than 65 N/20 mm, more preferably greater than 70 N/20 mm, more preferably greater than 75 N/20 mm, more preferably greater than 80 N/20 mm.

Item 27. The wound care product of any one of the preceding Items, wherein the free swell absorptive capacity of the absorbent body is higher than the free swell absorptive capacity of the porous cover, wherein the free swell absorptive capacity is determined according to EN 13726:2023-12.

Item 28. The wound care product of Item 27, wherein the free swell absorptive capacity of the absorbent body is at least 50% greater, preferably at least 80% greater, more preferably at least 100% greater, more preferably at least 200% greater, more preferably at least 500% greater, than the free swell absorptive capacity of the porous cover, wherein the free swell absorptive capacity is determined according to EN 13726:2023-12.

Item 29. The wound care product of any one of the preceding Items, wherein the fluid retention capacity of the absorbent body is greater than the fluid retention capacity of the porous cover, wherein the fluid retention capacity is determined according to EN 13726:2023-12.

Item 30. The wound care product of Item 29, wherein the fluid retention capacity of the absorbent body is at least 1.5 times the fluid retention capacity of the porous cover, preferably at least 1.8 times, more preferably at least 2 times, more preferably at least 3 times, more preferably at least 5 times.

Item 31. The wound care product of any one of the preceding Items, wherein the porous cover has a length-to-width ratio of at least 5, wherein the length is defined as the longest dimension of said porous cover, and the width is defined as the longest dimension perpendicular to said length.

Item 32. The wound care product of any one of the preceding Items, wherein the porous cover has a length-to-width ratio of at least 6, preferably at least 7, more preferably at least 8, more preferably at least 9, more preferably at least 10.

Item 33. The wound care product of any one of the preceding Items, wherein the width of the porous cover is greater than the width of the absorbent body.

Item 34. The wound care product of any one of the preceding Items, wherein the width of the porous cover is greater than 0.5 cm, preferably greater than 0.6 cm, more preferably greater than 0.7 cm, more preferably greater than 0.8 cm, more preferably greater than 0.9 cm.

Item 35. The wound care product of any one of the preceding Items, wherein the width of the porous cover is smaller than 5 cm, preferably smaller than 4 cm, more preferably smaller than 3 cm, more preferably smaller than 2.5 cm.

Item 36. The wound care product of any one of the preceding Items, wherein the width of the porous cover is greater than 0.5 cm and smaller than 5 cm, preferably greater than 0.5 cm and smaller than 4 cm, more preferably greater than 0.5 cm and smaller than 3 cm, more preferably greater than 0.5 cm and smaller than 2.5 cm, more preferably greater than 0.6 cm and smaller than 2.5 cm, more preferably greater than 0.7 cm and smaller than 2.5 cm, more preferably greater than 0.8 cm and smaller than 2.5 cm.

Item 37. The wound care product of any one of the preceding Items, wherein the porous cover has a length of more than 5 cm, preferably more than 10 cm, more preferably more than 15 cm, more preferably more than 20 cm, more preferably more than 25 cm, more preferably more than 30 cm, more preferably more than 35 cm, more preferably more than 40 cm.

Item 38. The wound care product of any one of the preceding Items, wherein the length of the porous cover is greater than the length of the absorbent body.

Item 39. The wound care product of any one of the preceding Items, wherein the porous cover is a tubular structure having two opposite ends.

Item 40. The wound care product of Item 39, wherein the porous cover is open on one of its two ends and closed on the other one of its ends.

Item 41. The wound care product of Item 40, wherein the absorbent body extends through the open end, such that a portion of the absorbent body is provided within the porous cover and another portion of the absorbent body is provided outside the porous cover.

Item 42. The wound care product of any one of the preceding Items, wherein the absorbent body has two opposite ends and the porous cover surrounds at least one of the two opposite ends of the absorbent body and is closed around said one end.

Item 43. The wound care product of any one of the preceding Items, wherein at least 70 length% of the absorbent body, preferably at least 80 length%, more preferably at least 90 length%, more preferably at least 95 length%, are surrounded by the porous cover, wherein "length%" is calculated as follows:

length% = (length of absorbent body surrounded by porous cover / total length of absorbent body) * 100%.

Item 44. The wound care product of any one of Items 1-40 and 42, wherein the entire absorbent body is surrounded by the porous cover such that the entire absorbent body is provided within the porous cover.

Item 45. The wound care product of any one of Items 39, 42, 43, and 44 wherein both ends of the porous cover are closed.

Item 46. The wound care product of any one of the preceding Items, wherein the wound care product comprises not more than one porous cover.

Item 47. The wound care product of any one of the preceding Items, wherein the porous cover is non-gelling such that the porous cover does not form a hydrogel upon contact with an aqueous liquid.

Item 48. The wound care product of any one of the preceding Items, wherein the absorbent body is more hydrophilic than the porous cover, wherein "hydrophilicity" is determined based on the contact angle determined according to ASTM D7334-08 (Reapproved 2022).

EP 4 717 238 A1

Item 49. The wound care product of any one of the preceding Items, wherein the porous cover is made of a material that defines a contact angle of at least 80 degrees, preferably at least 90 degrees, more preferably at least 100 degrees, more preferably at least 110 degrees, wherein the contact angle is determined according to ASTM D7334-08.

Item 50. The wound care product of any one of the preceding Items, wherein the porous cover comprises or consists of a member selected from the list consisting of nylon; viscose; polyester such as polyethylene terephthalate (PET), polybutylene terephthalate (PBT), and polytrimethylene terephthalate (PTT); polyethylene; polypropylene; acrylic fibers; olefin fibers; polytetrafluoroethylene-coated materials; silicone-coated materials, preferably silicone-coated viscose; materials coated with polyvinylchloride or with other vinyl-based materials; and polyamide.

Item 51. The wound care product of any one of the preceding Items, wherein the porous cover is made of a fibrous material.

Item 52. The wound care product of Item 51, wherein the fibrous material is a yarn.

Item 53. The wound care product of any one of the preceding Items, wherein the porous cover is a knitted structure or a woven structure.

Item 54. The wound care product of any one of claims 1-51, wherein the porous cover is a welded structure or a non-woven structure

Item 55. The wound care product of any one of the preceding Items, wherein size of the pores of the porous cover (also referred to herein as "pore size") is smaller than the smallest dimension of the absorbent body.

Item 56. The wound care product of any one of the preceding Items, wherein the pores have an average size of 0.15 mm to 5 mm, preferably 0.17 mm to 4 mm, more preferably 0.17 mm to 3 mm, more preferably 0.3 mm to 3 mm, more preferably 0.3 mm to 2.5 mm, more preferably 0.5 mm to 2.5 mm, more preferably 1 mm to 2 mm.

Item 57. The wound care product of any one of the preceding Items, wherein the porous cover has a uniform material composition, wherein uniform material composition means that the material properties of the porous cover do not vary in terms of composition and vary not by more than 5%, preferably not by more than 1%, in terms of physical parameters characterizing the material across the entire area of the porous cover.

Item 58. The wound care product of any one of the preceding Items, wherein the width of the porous cover is greater than the width of the absorbent body, preferably wherein the width of the porous cover is greater than the width of the absorbent body and length of the porous cover is greater than the length of the absorbent body.

Item 59. The wound care product of any one of the preceding Items, wherein the wound care product has a re-wetting capability of 0.1 g or more, preferably 0.2 g or more, as determined by the re-wetting test described in the description.

**Examples**

The present invention is now further illustrated by the following examples.

**<u>Wet tensile strength</u>**

Different non-gelling yarns have been used to produce textile porous covers (circular knit). Then, a PVA absorbent body (2 x 45 cm) was introduced into the textile porous covers. The wet tensile strength was measured in accordance with EN 29073-3:1992 as described above. 2 (Samples 1-4) or 3 (Sample 5) measurements were performed on each sample and the results are the averages of these 2 or 3 measurements. Fig. 7 shows an inventive wound care product during the measurement of the wet tensile strength.

The following materials were used for preparing the textile porous covers:

| Sample # | Material |
| --- | --- |
| 1 | Nylon/silicone coating (45% nylon, 55% silicone), 260 dtex |
| 2 | Viscose 1/26ne |

(continued)

| Sample # | Material |
|---|---|
| 3 | Viscose 1/40ne |
| 4 | Nylon 1/78/34 dtex |
| 5 (comparative) | PVA absorbent body without porous cover |
| "ne" stands for 'new english/cotton count'<br>"dtex" stands for "decitex" | |

The results are shown below:

| Sample # | Wet tensile strength (N/20 mm) |
|---|---|
| 1 | Very high; not determined because the tensile strength is so high that the sample slips out of the sample holder before it ruptures. |
| 2 | 58.45 |
| 3 | 32.09 |
| 4 | 168.10 |
| 5 (comparative) | 5.71 |

It can be seen that the provision of the porous cover in all cases significantly increased the tensile strength of the wound care product.

**Re-wetting capability**

The re-wetting behavior of the samples has been determined according to the following re-wetting test:

1) About 10cm of product was put on a double folded pre-weighed copy paper.

2) A plexiglass plate and weights to equal about 40 mmHg was added (about 830g: 1.5x10x40x1.36) on top for about 2 min.

3) The plexiglass, the weights, and the sample were removed from the paper and the paper was weight again.

The results are summarized in the following table:

| Sample | Specimen number | Weight (g) | | | Mean weight of absorbed water (g) |
|---|---|---|---|---|---|
| | | Copy paper | Wet Copy paper | Absorbed water | |
| 1 | 1 | 6,2 | 6,37 | 0,17 | |
| | 2 | 6,25 | 6,35 | 0,10 | **0,135** |
| 2 | 1 | 6,21 | 6,66 | 0,45 | |
| | 2 | 6,16 | 6,65 | 0,49 | **0,470** |
| 3 | 1 | 6,13 | 6,52 | 0,39 | |
| | 2 | 6,16 | 6,64 | 0,48 | **0,435** |
| 4 | 1 | 6,3 | 6,73 | 0,43 | |
| | 2 | 6,23 | 6,61 | 0,38 | **0,405** |
| 5 | 1 | 6,16 | 6,45 | 0,29 | |
| | 2 | 6,28 | 6,53 | 0,25 | **0,270** |

It can be seen that the re-wetting capability can be fine-tuned by modifying the material of the porous cover. For example, the most hydrophobic porous cover (silicone-coated nylon) has the lowest re-wetting capability. Further, all samples have a re-wetting capability of more than 0.1 g. Further, the re-wetting capability of all samples is in the same range as the re-wetting capability of a PVA absorbent body (Sample 5) which is known to have a re-wetting capability desired for wound treatment.

## Claims

1.  Wound care product, comprising:

    a) an absorbent body,

       wherein said absorbent body comprises absorbent fibers,
       wherein said absorbent body has a length-to-width ratio of at least 5, wherein the length is defined as the longest dimension of said absorbent body, and the width is defined as the longest dimension perpendicular to said length; and

    b) a porous cover surrounding at least a part of said absorbent body.

2.  The wound care product of claim 1, **characterized in that** it is a wound dressing, preferably a ribbon dressing or a rope dressing

3.  The wound care product of any one of the preceding claims, wherein the absorbent fibers are gelling absorbent fibers, i.e. form a hydrogel upon contact with aqueous liquids.

4.  The wound care of any one of the preceding claims, wherein the absorbent fibers comprise or consist of a member selected from the group consisting of polyacrylates, polyvinyl alcohol (PVA), preferably cross-linked PVA; polysaccharides, preferably modified cellulose, more preferably carboxymethyl cellulose (CMC); polyurethane polymer with polyethylene glycol and/or polypropylene functionalities; alginate; and copolymers thereof.

5.  The wound care product of claim 4, wherein the gelling absorbent fibers comprise or consist of polyvinyl alcohol (PVA), preferably cross-linked PVA, CMC, or polyacrylate, most preferably PVA.

6.  The wound care product of any one of the preceding claims, wherein the wet tensile strength of the porous cover is higher than the wet tensile strength of the absorbent body, wherein the wet tensile strength is determined as described in the description, preferably:
    the wet tensile strength of the porous cover is at least 1.5 times the wet tensile strength of the absorbent body, preferably at least 2 times, more preferably at least 3 times, more preferably at least 4 times, more preferably at least 5 times, more preferably at least 6 times, more preferably at least 7 times, more preferably at least 8 times, more preferably at least 9 times, more preferably at least 10 times.

7.  The wound care product of any one of the preceding claims, wherein the free swell absorptive capacity of the absorbent body is higher than the free swell absorptive capacity of the porous cover, wherein the free swell absorptive capacity is determined according to EN 13726:2023-12, preferably:
    the free swell absorptive capacity of the absorbent body is at least 50% greater, preferably at least 80% greater, more preferably at least 100% greater, more preferably at least 200% greater, more preferably at least 500% greater, than the free swell absorptive capacity of the porous cover, wherein the free swell absorptive capacity is determined according to EN 13726:2023-12.

8.  The wound care product of any one of the preceding claims, wherein the fluid retention capacity of the absorbent body is greater than the fluid retention capacity of the porous cover, wherein the fluid retention capacity is determined according to EN 13726:2023-12, preferably:
    the fluid retention capacity of the absorbent body is at least 1.5 times the fluid retention capacity of the porous cover, preferably at least 1.8 times, more preferably at least 2 times, more preferably at least 3 times, more preferably at least 5 times.

9.  The wound care product of any one of the preceding claims, wherein the porous cover is a tubular structure having two opposite ends.

10. The wound care product of any one of the preceding claims, wherein the entire absorbent body is surrounded by the porous cover such that the entire absorbent body is provided within the porous cover.

11. The wound care product of any one of the preceding claims, wherein the porous cover is non-gelling such that the porous cover does not form a hydrogel upon contact with an aqueous liquid.

12. The wound care product of any one of the preceding claims, wherein the absorbent body is more hydrophilic than the porous cover, wherein "hydrophilicity" is determined based on the contact angle determined according to ASTM D7334-08 (Reapproved 2022).

13. The wound care product of any one of the preceding claims, wherein the porous cover comprises or consists of a member selected from the list consisting of nylon; viscose; polyester such as polyethylene terephthalate (PET), polybutylene terephthalate (PBT), and polytrimethylene terephthalate (PTT); polyethylene; polypropylene; acrylic fibers; olefin fibers; polytetrafluoroethylene-coated materials; silicone-coated materials, preferably silicone-coated viscose; materials coated with polyvinylchloride or with other vinyl-based materials; and polyamide.

14. The wound care product of any one of the preceding claims, wherein the porous cover is a knitted structure or a woven structure.

15. The wound care product of any one of the preceding claims, wherein the pores have an average size of 0.15 mm to 5 mm, preferably 0.17 mm to 4 mm, more preferably 0.17 mm to 3 mm, more preferably 0.3 mm to 3 mm, more preferably 0.3 mm to 2.5 mm, more preferably 0.5 mm to 2.5 mm, more preferably 1 mm to 2 mm.

Fig. 1a

Fig. 1b

*Fig. 1c*

*Fig. 2*

*Fig. 3*

2.5 cm

10 cm

7.5 cm

*Fig. 4*

Fig. 5

Fig. 6

Fig. 7

Europäisches
Patentamt

European
Patent Office

Office européen
des brevets

# EUROPEAN SEARCH REPORT

**Application Number**

EP 24 20 3427

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 7 838 719 B2 (HILTON JIMMY E JR [US]) 23 November 2010 (2010-11-23) | 1,2,7-13 | INV. A61F13/01 |
| Y | * figures 1-1F * <br> * figure 3 * <br> * column 7, lines 63-65 * <br> * column 9, lines 52-54 * | 14,15 | A61F13/36 <br> A61F15/00 <br> A61L15/24 |
| Y | US 2014/257221 A1 (KETTLEWELL GRAEME [GB] ET AL) 11 September 2014 (2014-09-11) <br> * paragraph [0005] * | 1-15 | |
| Y | Anonymous: "Gelling Fiber Dressing", <br> , <br> 1 January 2015 (2015-01-01), XP055407472, <br> Retrieved from the Internet: <br> URL:http://www.molnlycke.co.uk/PageFiles/6 6712/Exufiber Product Sheet.pdf <br> [retrieved on 2017-09-18] <br> * the whole document * | 1-15 | |
| Y | EP 0 594 034 A1 (INT VERBANDSTOFF SCHAFFHAUSEN [CH]) 27 April 1994 (1994-04-27) <br> * column 2, lines 23-34; figure 10 * | 14,15 | **TECHNICAL FIELDS SEARCHED (IPC)** <br><br> A61F <br> A61L |
| A | WO 90/01913 A1 (FLAWA SCHWEIZ VERBAND WATTEFAB [CH]) 8 March 1990 (1990-03-08) <br> * the whole document * | 1-15 | |
| A | US 11 992 390 B2 (ADV MED SOLUTIONS LTD [GB]) 28 May 2024 (2024-05-28) <br> * the whole document * | 1-15 | |

The present search report has been drawn up for all claims

| Place of search | Date of completion of the search | Examiner |
|---|---|---|
| Munich | 13 February 2025 | Beckert, Audrey |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P4C01)

## ANNEX TO THE EUROPEAN SEARCH REPORT
## ON EUROPEAN PATENT APPLICATION NO.

EP 24 20 3427

This annex lists the patent family members relating to the patent documents cited in the above-mentioned European search report.
The members are as contained in the European Patent Office EDP file on
The European Patent Office is in no way liable for these particulars which are merely given for the purpose of information.

13-02-2025

| Patent document cited in search report | | | Publication date | Patent family member(s) | | Publication date |
|---|---|---|---|---|---|---|
| US 7838719 | B2 | | 23-11-2010 | NONE | | |
| US 2014257221 | A1 | | 11-09-2014 | CN | 104027832 A | 10-09-2014 |
| | | | | EP | 2774629 A2 | 10-09-2014 |
| | | | | GB | 2511528 A | 10-09-2014 |
| | | | | US | 2014257221 A1 | 11-09-2014 |
| EP 0594034 | A1 | | 27-04-1994 | AT | E147253 T1 | 15-01-1997 |
| | | | | DK | 0594034 T3 | 07-07-1997 |
| | | | | EP | 0594034 A1 | 27-04-1994 |
| | | | | HK | 1001225 A1 | 05-06-1998 |
| WO 9001913 | A1 | | 08-03-1990 | EP | 0383886 A1 | 29-08-1990 |
| | | | | WO | 9001913 A1 | 08-03-1990 |
| US 11992390 | B2 | | 28-05-2024 | EP | 3666238 A1 | 17-06-2020 |
| | | | | GB | 2579790 A | 08-07-2020 |
| | | | | US | 2020188179 A1 | 18-06-2020 |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20130323195 A
- US 20130274415 A

- WO 2013041620 A

**Non-patent literature cited in the description**

- **TIMMONS** ; **COOPER**. How to systematically assess a patient with a cavity wound. *Wounds UK*, 2008, vol. 4 (2), 4-10
- **VOWDEN K**. Defining, assessing and managing cavity wounds. *Wounds UK*, 2016, vol. 12 (1), 18-23

- **GEFEN A**. How medical engineering has changed our understanding of chronic wounds and future prospects. *Med Eng Phys*, 2019, vol. 72, 13-18
- **LUSTIG A** ; **ALVES P** ; **CALL E et al.** The sorptivity and durability of gelling fibre dressings tested in a simulated sacral pressure ulcer system. *Int Wound J*, 2021, vol. 18 (2), 194-208